Europäisches Patentamt

European Patent Office

Office européen des brevets

(11) Numéro de publication : **0 052 862**
**B1**

# FASCICULE DE BREVET EUROPÉEN

(45) Date de publication du fascicule du brevet :
**13.06.84**

(21) Numéro de dépôt : **81109771.6**

(22) Date de dépôt : **19.11.81**

(51) Int. Cl.³ : **A 61 L  2/02,** A 23 L  3/00,
A 23 C  7/04, B 01 D 31/00,
B 01 D 13/00

(54) **Dispositif de stérilisation d'un liquide.**

(30) Priorité : **26.11.80 FR 8025059**

(43) Date de publication de la demande :
**02.06.82 Bulletin 82/22**

(45) Mention de la délivrance du brevet :
**13.06.84 Bulletin 84/24**

(84) Etats contractants désignés :
**BE CH DE FR GB IT LI NL SE**

(56) Documents cités :
**FR-A- 1 387 643**
**FR-A- 2 160 853**
**FR-A- 2 251 351**
**FR-A- 2 473 313**

(73) Titulaire : **COMPAGNIE GENERALE D'ELECTRICITE**
**Société anonyme dite:**
**54, rue La Boétie**
**F-75382 Paris Cedex 08 (FR)**

(72) Inventeur : **Galaj, Stanislas**
**27, avenue Lénine**
**F-94110 Arcueil (FR)**
Inventeur : **Petitbon, Alain** ·
**2, avenue de l'Essonne**
**F-91130 Ris-Orangis (FR)**

(74) Mandataire : **Weinmiller, Jürgen et al**
**Zeppelinstrasse 63**
**D-8000 München 80 (DE)**

Jouve, 18, rue St-Denis, 75001 Paris, France

**Description**

La présente invention concerne un dispositif de stérilisation d'un liquide.

On connaît un dispositif de stérilisation d'un liquide décrit dans la demande de brevet français publiée sous le numéro 2.473.313.

Ce dispositif comprend :

— un filtre tubulaire cylindrique poreux en céramique, ce filtre étant formé d'une part d'un support tubulaire extérieur dont les dimensions des pores sont comprises entre 1 et 10 microns et d'autre part d'une membrane tubulaire intérieure solidaire de la surface cylindrique intérieure du support, les pores de cette membrane ayant une dimension comprise entre 0,1 et 0,22 micron de façon à retenir les bactéries contenues dans le liquide,

— un circuit de pompage relié au filtre pour faire passer le liquide dans le volume intérieur du filtre d'une première de ses extrémités à l'autre,

— un circuit de recyclage, en série avec le circuit de pompage, apte à renvoyer vers la première extrémité du filtre le liquide sortant de la deuxième extrémité,

— des moyens pour maintenir la pression du liquide en circulation à une valeur fixe prédéterminée suffisamment élevée pour qu'une partie du liquide traverse la paroi du filtre

— et un récipient disposé pour recevoir ladite partie du liquide ainsi stérilisée.

Dans le cas où le liquide à stériliser est par exemple du lait écrémé, on constate que le dispositif connu décrit ci-dessus présente l'inconvénient d'entraîner un colmatage relativement rapide du filtre lorsque la vitesse d'écoulement du lait le long de la membrane est faible, par exemple inférieure à 0,5 m/s, ou lorsque la pression du lait est trop élevée, par exemple supérieure à 5 bars.

La présente invention a pour but de pallier cet inconvénient.

La présente invention a pour objet un dispositif de stérilisation d'un liquide, comprenant :

— un filtre tubulaire cylindrique poreux en céramique, ce filtre étant formé d'une part d'un support tubulaire extérieur dont les dimensions des pores sont comprises entre 1 et 10 microns (μm) et d'autre part d'une membrane tubulaire intérieure solidaire de la surface cylindrique intérieure du support, les pores de cette membrane ayant une dimension comprise entre 0,1 et 0,22 microns (μm) de façon à retenir les bactéries contenues dans le liquide,

— un circuit de pompage relié au filtre pour faire passer le liquide dans le volume intérieur du filtre d'une première de ses extrémités à l'autre,

— un circuit de recyclage, en série avec le circuit de pompage, apte à renvoyer vers la première extrémité du filtre le liquide sortant de la deuxième extrémité,

— des moyens pour maintenir la pression du liquide en circulation à une valeur fixe prédéterminée suffisamment élevée pour qu'une partie du liquide traverse la paroi du filtre

— et un récipient disposé pour recueillir ladite partie du liquide ainsi stérilisée,

caractérisé en ce que le filtre comporte en outre un noyau sensiblement cylindrique disposé coaxialement dans le volume intérieur du filtre, de façon à canaliser le liquide dans l'espace tubulaire compris entre la surface cylindrique du noyau.

Des formes particulières d'exécution de l'objet de la présente invention sont décrites ci-dessous, à titre d'exemple, en référence aux dessins annexés, dans lesquels

la figure 1 représente schématiquement un mode de réalisation du dispositif selon l'invention,

la figure 1A représente à échelle agrandie la partie A de la figure 1, et la figure 2 est une coupe longitudinale d'un mode de réalisation d'un élément de filtrage faisant partie du dispositif illustré par la figure 1.

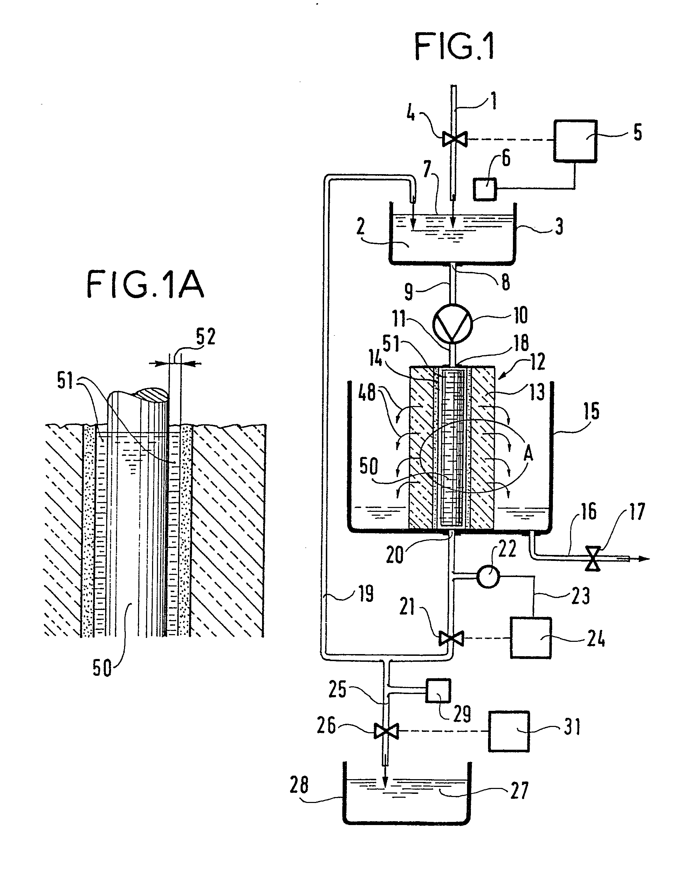

Sur la figure 1, l'extrémité d'une canalisation 1 est disposée au-dessus d'un liquide 2 contenu dans une cuve 3. La canalisation 1 comporte une vanne 4 dont l'ouverture et la fermeture est commandée par un système d'asservissement 5. Le système 5 est relié à un capteur 6 du niveau d'équilibre 7 du liquide 2.

La cuve 3 comporte une ouverture inférieure 8 reliée par un circuit de pompage à une extrémité 18 du volume intérieur d'un tube 12. Ce circuit comporte en série une canalisation 9, une pompe 10 et une canalisation 11.

Le tube 12 est formé d'un support tubulaire macroporeux 13 et d'une membrane microporeuse 14 déposée sur la surface cylindrique intérieure du support 13. Le support 13 est constitué d'une céramique poreuse qui peut être par exemple de l'alumine. Les pores de la céramique constituant le support 13 ont une dimension comprise entre 1 et 10 microns (μm). L'épaisseur de ce support peut être comprise entre 0,5 et 2 mm, et son diamètre est de l'ordre de 1 cm. La membrane 14 est formée aussi d'une céramique poreuse qui peut comprendre par exemple de l'alumine, de la zircone ou de la silice. Les pores de la membrane 14 ont une dimension comprise entre 0,1 et 0,22 micron (μm) et son épaisseur peut être comprise entre 10 et 50 microns (μm).

La surface cylindrique extérieure du tube 12 est entourée par un récipient cylindrique coaxial 15. Une canalisation 16 munie d'une vanne 17 est reliée à une ouverture inférieure du récipient 15.

Dans le volume intérieur du tube 12 est disposé coaxialement un noyau métallique 50, sensiblement cylindrique, dont la surface cylindrique délimite, avec la surface cylindrique intérieure du tube 12, un espace tubulaire 51 dont l'épaisseur radiale 52 (figure 1A) est faible par rapport au diamètre du tube 12. A titre d'exemple, pour un tube 12 de 1 cm de diamètre, l'épaisseur 52 peut être de 1 mm.

Un circuit de recyclage est constitué par une canalisation 19 partant de l'autre extrémité 20 du volume intérieur du tube 12 et aboutissant au-dessus de la surface d'équilibre 7 du liquide contenu dans la cuve 3.

Une vanne 21 est disposée sur la canalisation 19 à la sortie du tube 12. un manomètre 22 est disposé en dérivation sur la canalisation 19 entre l'extrémité 20 et la vanne 21. L'information de la pression lue par le manomètre 22 est transmise par une connexion électrique 23 à un système d'asservissement 24 commandant l'ouverture et la fermeture de la vanne 21.

Une canalisation 25 est branchée en dérivation sur la canalisation 19 à la sortie de la vanne 21. La canalisation 25 est munie d'une vanne 26 et aboutit au-dessus du niveau d'équilibre d'un liquide contenu dans une cuve 28. Un appareil 29 est branché en dérivation sur la canalisation 25 pour effectuer des prélèvements périodiques du liquide en circulation. Un système 31 commande l'ouverture et la fermeture de la vanne 26.

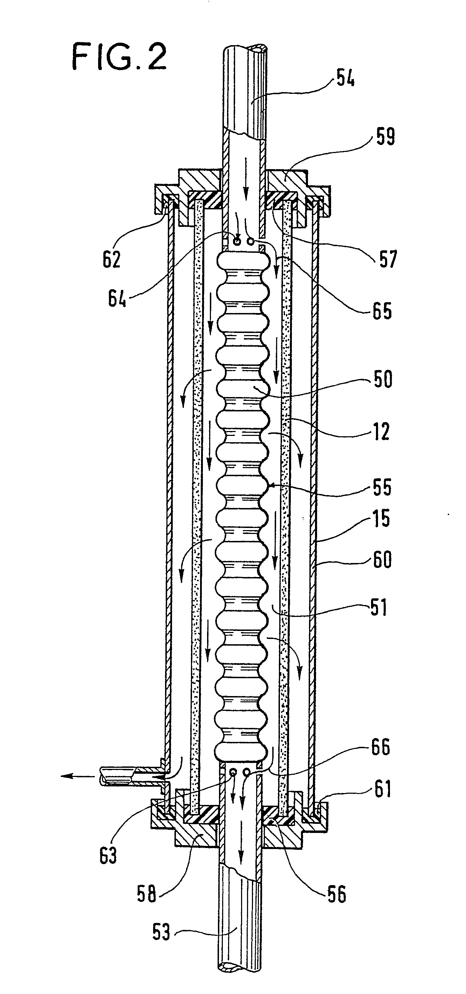

La figure 2 montre en coupe, un mode de réalisation de l'élément de filtrage, qui comporte essentiellement le tube 12, le noyau 50 et le récipient 15.

Comme il est visible sur la figure 2, deux tubes métalliques 53 et 54 sont fixés coaxialement sur les deux faces extrêmes du noyau 50 en prolongement de ce noyau.

La surface cylindrique du noyau 50 est annelée, c'est-à-dire que sa section axiale n'est pas rigoureusement rectiligne comme représenté sur les figures 1 et 1A, mais présente des ondulations successives 55 s'échelonnant sur la longueur du noyau d'une face extrême à l'autre.

Un joint torique 56 monté sur la surface extérieure du tube 53 s'appuie sur l'extrémité du tube 12 disposée en regard grâce à un serre-joint 58. De même, un joint torique 57 monté sur la surface extérieure du tube 54 s'appuie sur l'autre extrémité du tube 12 grâce à un serre-joint 59.

La paroi du tube 53 comporte des ouvertures 63 disposées entre le joint 56 et la face extrême du noyau 50 en contact avec ce tube, pour mettre en communication l'espace 51 avec le volume interne du tube 53. De même, la paroi du tube 54 comporte des ouvertures 64 disposées entre le joint 57 et l'autre face extrême du noyau 50, pour mettre en communication l'espace 51 avec le volume interne du tube 54.

La paroi du réservoir 15 comporte un tube cylindrique 60 qui s'appuie sur les serre-joints 58 et 59 par l'intermédiaire de joints toriques 61 et 62.

Le dispositif de stérilisation décrit ci-dessus et représenté sur les figures 1 et 2 fonctionne de la manière suivante.

Le liquide à stériliser, qui peut être par exemple du lait écrémé, arrive par la canalisation 1. Lorsque la vanne 4 est ouverte, le lait sortant de la canalisation 1 s'écoule dans la cuve 3. La pompe 10 fait circuler le lait 2 remplissant la cuve 3 dans le volume intérieur du tube 12, de l'extrémité 18 à l'extrémité 20 de ce tube.

Dans le cas où l'élément de filtrage est tel que représenté sur la figure 2, le lait à filtrer arrive par le tube 54 et traverse les ouvertures 64 pour pénétrer suivant la direction 65 dans l'espace tubulaire 51, à une extrémité du tube 12. Il s'écoule ensuite parallèlement à l'axe du noyau 50 vers l'autre extrémité du tube 12 et traverse les ouvertures 63 pour pénétrer, suivant la direction 66, dans le tube 53 vers la canalisation de recyclage 19.

Grâce à la présence du noyau 50, le lait circulant dans le tube 12 est canalisé dans le mince espace tubulaire 51, ce qui a pour effet d'augmenter sa vitesse d'écoulement le long de la membrane 14. A titre d'exemple, avec une épaisseur 52 de un millimètre, on peut obtenir une vitesse d'écoulement de 1 m/s pour une pression inférieure à 5 bars. Dans ces conditions le tube 12 peut fonctionner pendant une période beaucoup plus longue, sans colmatage.

Ce résultat est atteint même si la surface latérale du noyau 50 est parfaitement cylindrique. Mais on utilise de préférence un noyau 50 annelé, comme représenté sur la figure 2, qui apporte des turbulences dans l'écoulement du lait et casse la couche limite devant la membrane en céramique, ce qui entraîne une amélioration de l'efficacité de la filtration.

La pression du lait circulant dans le tube 12 est lue par le manomètre 22, et le système d'asservissement 24, qui reçoit l'information de la pression mesurée par le manomètre 22, règle l'ouverture de la vanne 21 de façon à maintenir cette pression à une valeur fixe, suffisamment élevée pour qu'une partie du lait traverse radialement la paroi du tube 12 dans la direction indiquée par les flèches 48.

La membrane 14, dont la dimension des pores a été définie plus haut retient les bactéries mais laisse passer les matières organiques du lait, le support poreux 13 jouant ici un rôle purement mécanique, de façon à permettre au tube 12 de résister à la pression interne du lait.

La partie du lait qui a traversé les pores du tube 12 est ainsi stérilisée. Cette partie est recueillie dans le récipient 15 et peut être prélevée par ouverture de la vanne 17 en vue de son conditionnement.

La majeure partie du lait qui est entrée par l'extrémité 18 sort du tube 12 par l'extrémité 20 sans avoir traversé les pores du tube. Cette partie est renvoyée vers la cuve 3 par la canalisation 19. Le système d'asservissement 5, qui reçoit du capteur 6 l'information du niveau 7 du lait dans la cuve 3, règle l'ouverture de la vanne 4 de façon à maintenir ce niveau sensiblement constant.

Le volume de lait contenu dans le circuit fermé constitué par la cuve 3, la canalisation 9, la pompe 10, la canalisation 11, le tube 12 et la canalisation 19 reste donc constant, l'apport de lait par la canalisation 1 compensant la quantité de lait filtrée à travers les pores du tube 12. Il en résulte que ce volume s'enrichit progressivement en bactéries.

L'appareil 29 effectue des prélèvements de lait à intervalles de temps réguliers, le taux des bactéries contenues dans le liquide prélevé étant déterminé par exemple par une méthode optique de comptage en boîte de Pétri. Lorsque ce taux atteint un seuil prédéterminé, le système 31 permet de commander l'ouverture de la vanne 26, provoquant ainsi la vidange du circuit fermé dans la cuve 28. On peut aussi commander l'ouverture de la vanne 26 à des intervalles de temps prédéterminés au cours d'essais antérieurs, ces intervalles dépendant du type de lait à stériliser.

Le seuil prédéterminé est choisi, de préférence, à un niveau suffisamment bas pour éviter le colmatage des pores du tube 12.

Le support 13 du tube 12 peut être réalisé, par une méthode connue, par filage d'une pâte, le tube ainsi obtenu étant ensuite décrassé et fritté. La membrane 14 est alors déposée sur la surface cylindrique interne du support 13 par enduction d'une barbotine. Celle-ci est ensuite frittée pour obtenir des pores dont les dimensions sont situées dans la gamme étroite qui a été précisée plus haut.

Le dispositif selon l'invention peut être appliqué notamment à la stérilisation des produits liquides alimentaires tels que le lait, le vin ou les jus de fruits.

Dans le cas du lait en particulier, le dispositif selon l'invention permet d'effectuer la stérilisation sur le lieu de production, supprimant ainsi des transports coûteux.

Avec une membrane dont les pores ont une dimension voisine de 0,1 micron (μm) et pour des fortes pressions (supérieures à 3 bars), on peut obtenir une séparation des protéines par formation d'une couche limite qui constitue une membrane dynamique : le liquide filtré recueilli dans le récipient 15 contient alors la lactose et les protéines solubles, tandis que la partie du lait retenue par le filtre contient la caséine. La séparation ainsi réalisée permet l'obtention de caséine non dénaturée intéressante pour l'industrie fromagère et l'industrie des caséinates.

Avec une vitesse de circulation faible (par exemple 0,5 m/s), on obtient quasiment une ultrafiltration par renforcement de la membrane dynamique. Le liquide filtré recueilli dans le récipient 15 ne contient alors pratiquement plus de protéines.

## Revendications

1. Dispositif de stérilisation d'un liquide, comprenant
— un filtre tubulaire cylindrique poreux en céramique, ce filtre étant formé d'une part d'un support tubulaire extérieur dont les dimensions des pores sont comprises entre 1 et 10 microns (μm) et d'autre part d'une membrane tubulaire intérieure solidaire de la surface cylindrique intérieure du support, les pores de cette membrane ayant une dimension comprise entre 0,1 et 0,22 microns (μm) de façon à retenir les bactéries contenues dans le liquide,

— un circuit de pompage relié au filtre pour faire passer le liquide dans le volume intérieur du filtre d'une première de ses extrémités à l'autre,
— des moyens pour maintenir la pression du liquide en circulation à une valeur fixe prédéterminée suffisamment élevée pour qu'une partie du liquide traverse la paroi du filtre
— un circuit de recyclage, en série avec le circuit de pompage, apte à renvoyer vers la première extrémité du filtre le liquide sortant de la deuxième extrémité,
— et un récipient disposé pour recueillir ladite partie du liquide ainsi stérilisée,
caractérisé en ce que le filtre comporte en outre un noyau (50) sensiblement cylindrique disposé coaxialement dans le volume intérieur du filtre (12), de façon à canaliser le liquide dans l'espace tubulaire (51) compris entre la surface cylindrique intérieure du filtre et la surface cylindrique du noyau.

2. Dispositif selon la revendication 1, caractérisé en ce que la surface cylindrique du noyau (50) est annelée de façon que sa section axiale présente des ondulations (55) successives d'une extrémité à l'autre du noyau.

3. Dispositif selon la revendication 1, caractérisé en ce que la liaison entre le circuit de pompage et le filtre comporte
— deux tubes (54, 53) d'arrivée et de sortie du liquide, ces tubes étant solidaires du noyau (50) et disposés en prolongement de celui-ci respectivement de part et d'autre de ses extrémités,
— deux joints d'étanchéité (56, 57), un (56) de ces joints s'appuyant sur la surface extérieure d'un tube (53) et sur l'extrémité du filtre disposée en regard, l'autre joint (57) s'appuyant sur la surface extérieure de l'autre tube (54) et sur l'autre extrémité du filtre, la paroi de chaque tube comportant des ouvertures (63, 64) entre le joint d'étanchéité et l'extrémité du noyau en contact avec ce tube, ces ouvertures étant disposées pour mettre en communication ledit espace (51) avec le volume interne du tube.

## Claims

1. A device for the sterilisation of a liquid, comprising
— a tubular cylindrical porous filter of ceramic material, this filter being formed on the one hand of an outer tubular support, the pore dimensions of which are in the range 1 to 20 microns (μm) and on the other hand of an inner tubular membrane integral with the cylindrical inner surface of the support, the pores of this membrane having a dimension in the range 0,1 to 0,22 microns (μm) in order to retain the bacteria contained in the liquid,
— a pumping circuit connected to the filter and causing the liquid to flow in the inner volume of the filter from a first one of its ends to the other end,
— means for maintaining the pressure of the liquid in circulation at a fixed predetermined value which is high enough to cause part of the liquid to traverse the wall of the filter,
— a recycling circuit, in series with the pumping circuit and able to return the liquid coming from the second end to the first end of the filter,
— and a receptacle disposed to collect said part of the liquid thus sterilized,
characterized in that the filter further comprises an essentially cylindrical core (50) disposed coaxially in the inner volume of the filter (12), to convey the liquid in the tubular space (51) between the inner cylindrical surface of the filter and the cylindrical surface of the core.

2. A device according to claim 1, characterized in that the cylindrical surface of the core (50) is corrugated, so that its axial section presents successive ondulations (55) from one end to the other of the core.

3. A device according to claim 1, characterized in that the connection between the pumping circuit and the filter comprises
— two inlet and outlet pipes (54, 53) for the liquid, these pipes being integral with the core (50) and disposed in the prolongation of the latter respectively on one and the other of its ends,
— two sealing joints (56, 57), one (56) of these joints pressing against the outer surface of a pipe (53) and against the adjacent end of the filter, the other joint (57) pressing against the outer surface of the other pipe (54) and against the other end of the filter, the wall of each pipe comprising openings (63, 64) between the sealing joint and the end of the core which is in contact with this pipe, these openings being disposed in order to put into communication said space (51) with the inner volume of the pipe.

**Ansprüche**

1. Vorrichtung zur Sterilisation einer Flüssigkeit, mit
— einem rohrförmigen zylindrischen porösen Keramikfilter, das einerseits aus einem äußeren rohrförmigen Träger, dessen Porengrößen zwischen 1 und 10 Mikron (μm) liegen, und andererseits aus einer rohrförmigen inneren Membran besteht, die mit der inneren zylindrischen Oberfläche des Trägers fest verbunden ist, wobei die Poren dieser Membran einen Durchmesser von zwischen 0,1 und 0,22 Mikron (μm) aufweisen, um so die in der Flüssigkeit enthaltenen Bakterien zurückzuhalten,
— einem Pumpkreis, der mit dem Filter verbunden ist, um die Flüssigkeit im Innenraum des Filters von einem ersten seiner Enden zum anderen Ende zu bringen,
— Mitteln, um den Druck der zirkulierenden Flüssigkeit auf einem vorbestimmten festen Wert zu halten, der hoch genung ist, daß ein Teil der Flüssigkeit durch die Wand des Filters dringt,
— einem Rückspeisekreis in Serie mit dem Pumpkreis, der die vom zweiten Ende des Filters ausgehende Flüssigkeit wieder zum ersten Ende zurückschicken kann,
— und einem Behälter, der den so sterilisierten Teil der Flüssigkeit auffängt,
dadurch gekennzeichnet, daß das Filter außerdem einen Kern (50) aufweist, der im wesentlichen zylindrisch ist und koaxial im Innenvolumen des Filters (12) angeordnet ist, so daß er die Flüssigkeit in dem rohrförmigen Bereich (51) zwischen der zylindrischen inneren Oberfläche des Filters und der zylindrischen Oberfläche des Kerns kanalisiert.

2. Vorrichtung nach Anspruch 1, dadurch gekennzeichnet, daß die zylindrische Oberfläche des Kerns (50) gewellt ist, so daß sein Achsschnitt aufeinanderfolgende Wellen (55) von einem zum anderen Ende des Kerns aufweist.

3. Vorrichtung nach Anspruch 1, dadurch gekennzeichnet, daß die Verbindung zwischen dem Pumpkreis und dem Filter
— zwei Zufuhr- und Abflußrohre (54, 53) für die Flüssigkeit, die mit dem Kern (50) fest verbunden sind und in seiner Verlängerung an seinen beiden Enden angeordnet sind,
— zwei Dichtungen (56, 57), von denen eine (56) sich auf der äußeren Oberfläche eines Rohrs (53) und auf dem ihm gegenüberliegenden Ende des Filters abstützt, während die andere Dichtung (57) sich auf der äußeren Oberfläche des anderen Rohrs (54) und dem anderen Ende des Filters abstützt, wobei die Wand jedes Rohrs Öffnungen (63, 64) zwischen der Dichtung und dem mit diesem Rohr in Kontakt stehenden Ende des Kerns aufweist und diese Öffnungen so angeordnet sind, daß sie den Bereich (51) mit dem Innenraum des Rohrs verbinden.

# FIG.1

# FIG.1A

# FIG.2